# EUROPEAN PATENT SPECIFICATION

(11) **EP 1 957 143 B1**
(45) Date of publication and mention of the grant of the patent: **02.03.2011**
(21) Application number: 06786632.7
(22) Date of filing: 10.07.2006
(51) Int. Cl.: A61M 25/10, A61F 2/06

(54) **SELECTIVELY COATED MEDICAL BALLOONS**
SELEKTIV BESCHICHTETE MEDIZINISCHE BALLONS
BALLONS MÉDICAUX ENDUITS SÉLECTIVEMENT

(30) Priority: 07.12.2005 US 296680
(43) Date of publication of application: 20.08.2008
(73) Proprietor: Boston Scientific Limited, Hastings Christ Church (BB)
(72) Inventor: EIDENSCHINK, Tracee, Wayzata, Minnesota 55391 (US); RIZQ, Raed, Fridley, Minnesota 55432 (US); ELIZONDO, David, Champlin, Minnesota 55316 (US); WEBER, Jan, 6228 GJ Maastricht (NL); BARRY, Kevin, Buffalo, Minnesota 55313 (US)
(74) Representative: Schildberg, Peter
(86) International application number: PCT/US2006/026544
(87) International publication number: WO 2007/067215

(56) References cited:
- WO-A-01/45781
- WO-A-97/31674
- JP-A- 6 169 995
- US-A1- 2003 114 915

## Description

### FIELD OF THE INVENTION

The present invention relates to the field of catheter assemblies, more particularly, balloon catheters.

### BACKGROUND OF THE INVENTION

Balloon catheters, having expandable balloon members located at the distal end of the balloon catheter, are employed in a variety of medical procedures including as dilatation devices for compressing atherosclerotic plaque which results in a narrowing of the arterial lining, and for delivery and expansion of prosthetic devices such as stents, to a lesion site, i.e. vessel obstruction, within a body vessel.

One medical procedure where balloon catheters are employed is percutaneous transluminal coronary angioplasty, or balloon angioplasty, which is a minimally invasive, non-surgical means of treating peripheral and coronary arteries. This technique consists of inserting an uninflated balloon catheter into the affected artery. Dilation of the diseased segment of artery is accomplished by inflating the balloon which pushes the atherosclerotic lesion outward, thereby enlarging the arterial diameter. Angioplasty has become recognized as an efficient and effective method of opening stenoses in the vascular system. In the most widely used form of angioplasty, a balloon catheter is guided through the vascular system until the balloon, which is carried at the distal end of a catheter shaft, and which may carry an expandable stent, is positioned across the stenosis or lesion, i.e., vessel obstruction. The balloon is then inflated to apply pressure to the obstruction which is essentially remolded by pressing it against the inner wall of the vessel whereby the vessel is opened for improved flow. Due the expansion of the balloon, the stent, which is situated on the balloon, is also expanded and released to aid in support and/or repair of the vessel wall.

A stent is typically crimped onto the balloon while the balloon is in a folded/wrapped configuration. Once the site of deployment has been reached, the balloon is inflated, thereby expanding the stent. The balloon is then deflated wherein it rewraps and is then removed from the body vessel.

One feature which may be desirable to facilitate advancement of the catheter assembly through the body vessel, is to impart lubriciousness to the catheter surfaces, including to the expandable balloon member. One method of achieving this has been to apply a lubricious coating to the exterior surface of the balloon.

Increasing the lubriciousness of the balloon surface can also facilitate withdrawal of the balloon from a deployed, expanded stent.

One problem that can occur when an expandable balloon member with a lubricious surface is employed for delivery of a stent, however, is that during insertion, delivery through the vessel, and deployment of the stent at the lesion site, the stent may slip off of the balloon due to the lubricity of the surface. Such unplanned movement can negatively impact the ability to correctly position the stent at the lesion site.

WO 97/31674 discloses an expandable balloon catheter having a first exterior surface with a given coefficient of friction and a second exterior surface with a greater coefficient of friction. In a compact form the first exterior surface is exposed to produce one coefficient of a friction during transfer of the collapsed or uninflated balloon to and across a lesion. When inflated, the second surface dominates the first surface and produces a second coefficient of a friction. Coating for the first surface are based on hydrogel, silicone and hydrophilic oil materials.

The problem of the invention is to provide amcdical balloon having a lubricious surface which has minimal negative impact on securement of an intraluminal medical device to the expandable balloon member, but which facilitates balloon withdrawal after deployment of an intraluminal medical device, wherein said lubricious surface has a minimal negative impact on the mechanical properties of the balloon

The problem is solved by an expandable medical balloon according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### SUMMARY OF THE INVENTION

In one aspect, the present invention relates to a predetermined strategic arrangement of differentially lubricious surfaces on a balloon catheter, particularly on an expandable balloon member of a catheter delivery device. The strategic arrangement of lubricious surfaces can facilitate balloon withdrawal after deployment of a medical device, but may have little if any negative impact on securement of the medical device to the expandable balloon member during delivery of the device through a body vessel. Furthermore, the strategic placement of the lubricious coating can prevent unexpected movement of the intraluminal medical device on the expandable balloon member during delivery.

In one embodiment, the balloon is selectively treated to impart lubricity by imparting lubricity to the surface in those areas between the folds of the balloon, such that the treated areas are not in contact with the crimped stent until deployment. Upon deflation of the balloon, the lubricious area, or a portion thereof, remains exposed on the outside of the collapsed balloon.

The invention thereby facilitates insertion into a vessel and withdrawal of the expandable balloon member from the stent after the stent has been deployed and expanded. The strategic arrangement of lubricious surfaces decreases the likelihood that the stent will slip from the balloon surface.

These and other aspects, embodiments and advantages of the present invention will be apparent to those of ordinary skill in the art upon review of the Detailed Description and Claims to follow.

### BRIEF DESCRIPTION OF THE DRAWINGS

FIG. 1 is a perspective view of a balloon in a folded configuration.
FIG. 2 is radial cross-sectional view of a balloon having a folded configuration similar to that of FIG. 1 taken at section 2 in FIG. 1.
FIG. 2a is a radial cross-section view of a balloon similar to that shown in FIG. 2 showing an alternative distribution of coating.
FIG. 2b is a radial cross-section view of a balloon similar to that shown in FIGS. 2-2a more coating distributed on the unexposed inner surface of the balloon folds.
FIG. 3 is a radial cross-sectional view of a balloon similar to that shown in FIGS. 1 and 2 in an expanded state.
FIG. 3a is a radial cross-sectional view of a balloon similar to that shown in FIGS. 1-3 in an expanded state, illustrating an alternative distribution of coating.
FIG. 3b is a radial cross-sectional view of a balloon similar to that shown if FIG. 1-3a having more coating distributed on the balloon surface.
FIG. 3c is a radial cross-sectional representation of a balloon similar to that shown in FIGS. 1-3 in a deflated state.
FIG. 4 is a radial cross-sectional view of a balloon similar to that shown in FIGS. 1-3 in combination with a stent.
FIG. 5 is a radial cross-sectional view of a balloon similar to that shown in FIGS. 1, 2 and 3 in an expanded state.
FIG. 6 is a radial cross-sectional view of a balloon illustrating another folded configuration.
FIG. 7 is a perspective view of a balloon shown with three wings prior to folding.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

All published documents, including all US patent documents, mentioned anywhere in this application are hereby expressly incorporated herein by reference in their entirety. Any copending patent applications, mentioned anywhere in this application are also hereby expressly incorporated herein by reference in their entirety.

For the purposes of this disclosure, like reference numerals in the figures shall refer to like features unless otherwise indicated.

Depicted in the figures are various aspects of the invention. Elements depicted in one figure may be combined with, or substituted for, elements depicted in another figure as desired.

The medical balloons described herein, have a first deflated state in which the balloon has at least two folded wings. Due to the folding, the balloon surface area can be divided in an outer exposed surface and a complementary inner surface which is unexposed. The unexposed surface includes the inner surface of the folded wing, as well as the unfolded area of the balloon which does not form the wing, and which is also covered by the folded wing. The totality of the unexposed surface, including both wing and non-wing areas, shall be referred to herein, as the inner surface.

The inner surface includes a lubricious surface treatment. This configuration is suitable for medical device delivery wherein it is desired that the medical device come into contact with the outer surface of the wings, but not with the inner surface. The balloons further have an expanded state.

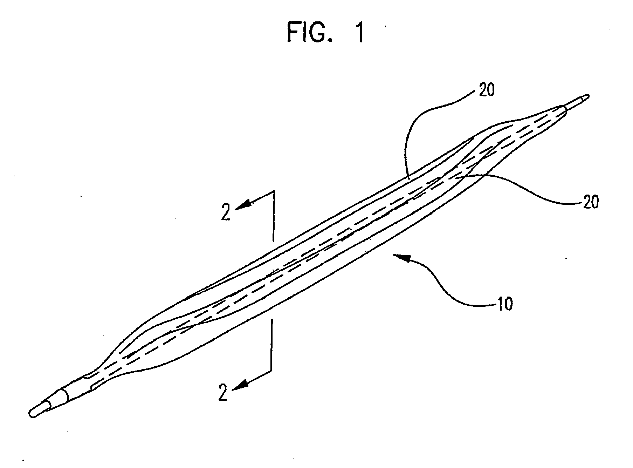

Turning now to the figures, FIG. 1 shows generally at 10, a balloon in a first deflated and folded configuration. After formation, a balloon may be aspirated by applying negative pressure to the balloon interior, causing it to deflate. The balloon, in this embodiment, has at least three folded wings 20.

Balloon 20, as shown in radial cross-section, taken at section 2 in FIG. 1, has a lubricious coating 30, shown on the inner surface 22 of wing 20. Wing 20 is folded such that the inner surface 22 of wing 20 is covered by the outer surface 24 of wing 20, such that when a medical device, such as a stent, is mounted thereon, the stent is in contact with the outer surface 24 of wing 20 in this first deflated configuration. This configuration may be employed during delivery of a stent to the lesion site.

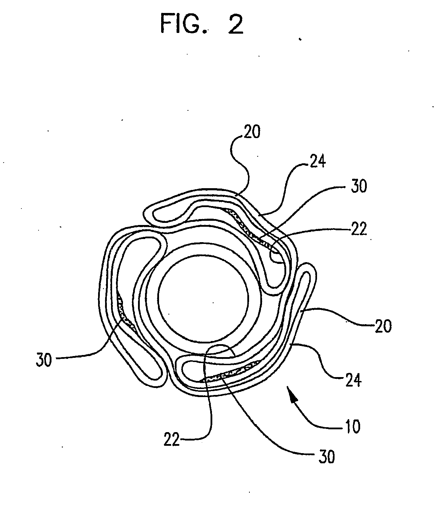

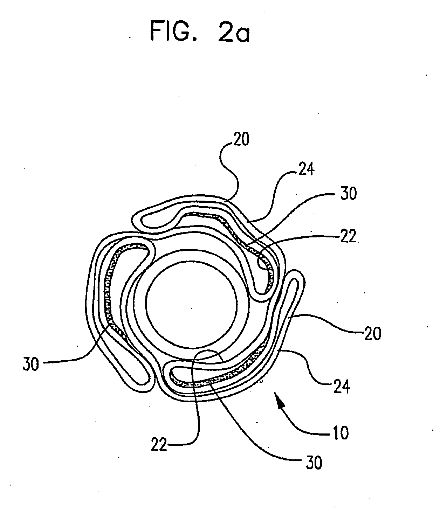

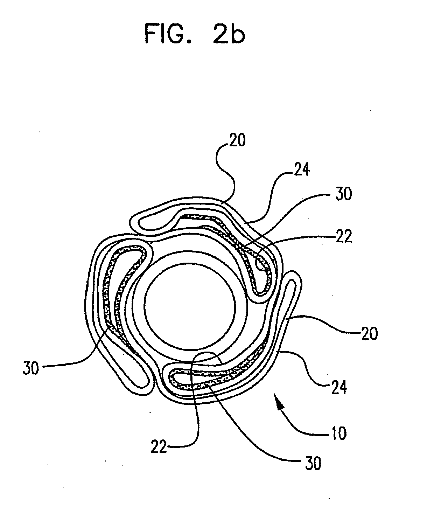

FIG. 2 is a radial cross-section of the balloon shown in FIG. 1 taken at section 2 in FIG. 1. FIG. 2a is a radial cross-section of a balloon similar to that shown in FIGS. 1 and 2, showing the lubricious coating 30 disposed on more of the balloon surface. Alternatively, the coating may also be distributed on a portion of the balloon surface which does not form a portion of the folded wing as shown in FIG. 2b.

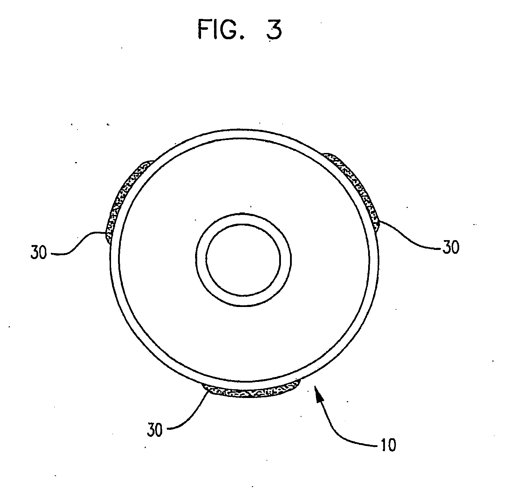

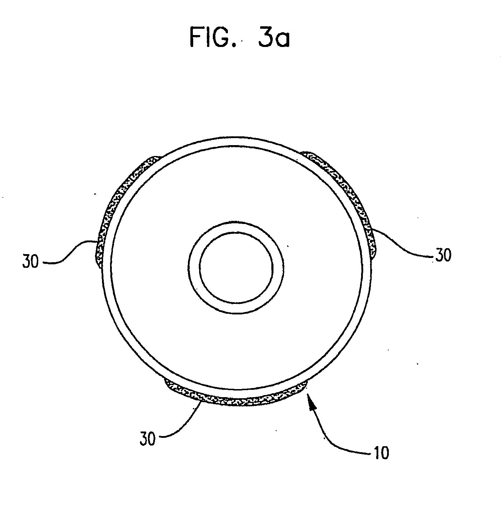

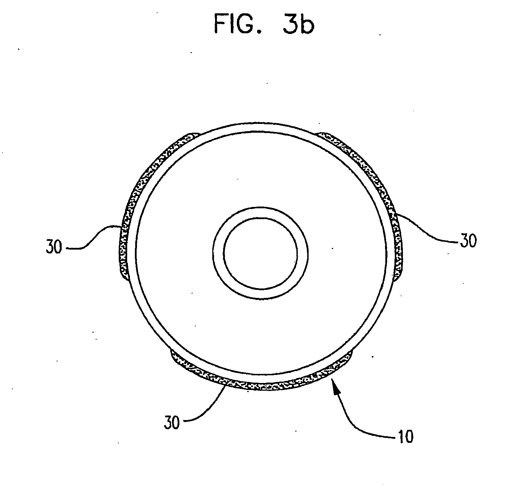

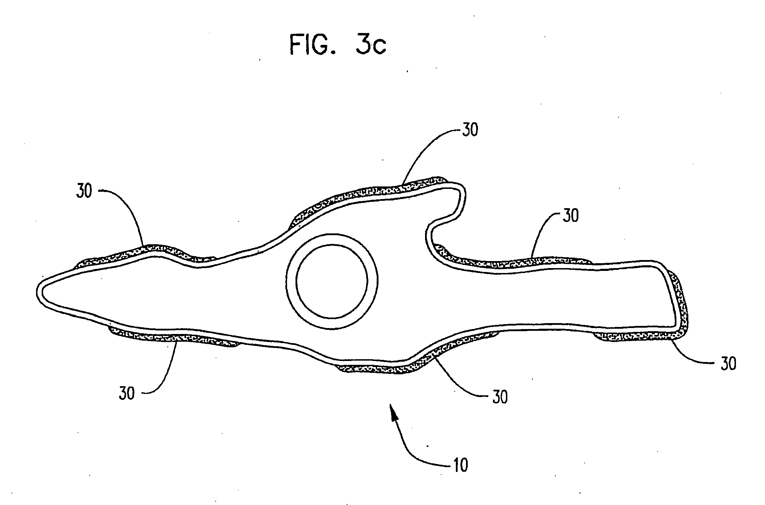

FIG. 3 is a radial cross-section of balloon 10 similar to that shown in FIGS. 1 and 2 in an expanded state showing lubricious coating 30, on the outer balloon surface.

FIG. 3a is a radial cross-section of balloon 10 similar to that shown in FIGS. 1-3 in an expanded state showing an alternative distribution of lubricious coating 30 wherein the lubricious coating is disposed on more of the balloon surface than in FIG. 3.

FIG. 3b is a radial cross-sectional view of balloon 10 similar to that shown in FIG. 1-3a wherein the lubricious coating is disposed over even more of the balloon surface.

Furthermore, it is possible for the lubricious coating to migrate or smear wherein the coating is transferred to the originally non-coated areas on the balloon surface during expansion and/or deflation resulting. FIG. 3c is a radial cross-sectional representation of a balloon similar to that shown in FIGS. 1-3 in a deflated state, illustrating smearing of the coating 30 on the balloon surface.

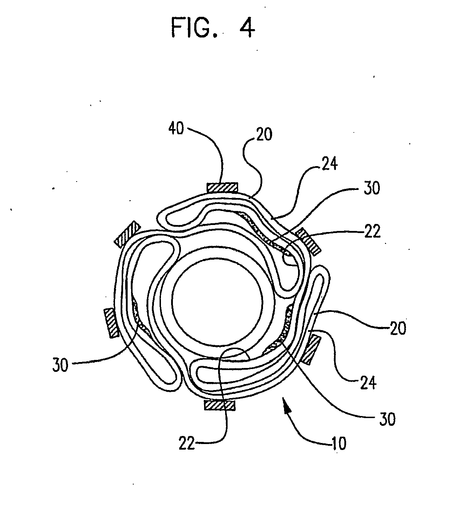

FIG. 4 is a radial cross-section of balloon 10, similar to the radial cross-section shown in FIGS. 1-2, but having a stent 40 mounted thereon. Stent 40 is shown in contact with the outer surface 24 of wing 20. The stent is not in contact with the lubriciously treated inner surface 22 during delivery through a patient's body vessel.

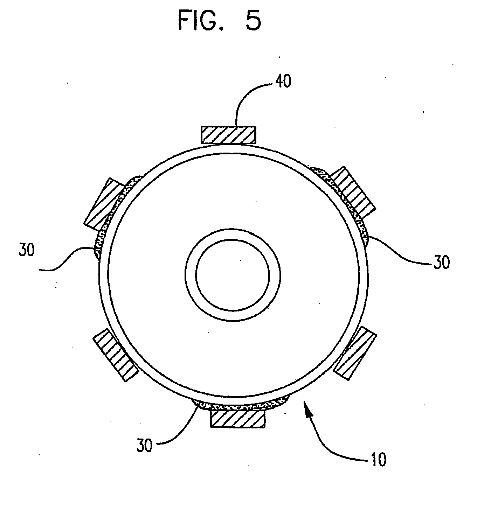

FIG. 5 is a radial cross-section of a balloon 10 similar to that in FIG. 4 in an expanded state. When inflated, the balloon, in this embodiment, assumes a cylindrical configuration.

After expansion of the balloon and stent, portions of the expanded balloon surface beneath the stent will be covered by the lubricious coating thereby decreasing the resistance during withdrawal of the catheter assembly after expansion and deployment of the stent.

Once the stent has been deployed, the balloon may be deflated by application of negative pressure. Deflation of the balloon after inflation permits reformation of wings or flaps which can become wrapped around the catheter shaft for further advancement of the catheter to treat another lesion, or withdrawal of the catheter from the patient. However, the balloon may not return to the exact same deflated configuration that it had prior to expansion. Consequently, the lubriciously treated surfaces may be at least partially exposed in the deflated state which occurs after balloon expansion.

The balloons according to the invention may be formed using any suitable balloon forming techniques. One method is to extrude a tube of polymer material, placing the extruded tube of polymer material in a mold form, and radially expanding the tube under pressure and with heat if required to soften the polymer material such that the mold form is filled and the polymer material takes on the shape of the mold form. Upon removal of the balloon from the mold form, the balloon is said to be in its static state. Such techniques are disclosed in U.S. Patent No. 4,490,421. The tube may also be axially stretched with positive pressure. Radial expansion caused by the positive pressure on the tube interior with axial stretching establishes biaxial stretching of the tube. The biaxial stretching is maintained until the tube assumes the shape of the mold.

In accordance with the present invention, a deflated dilatation balloon can be prepared for insertion through a patient's body vessel, such as through the cardiovascular system, by a series of steps to create folded wings or flaps. The wings may be wrapped circumferentially to provide a minimized outer diameter when the balloon is in its deflated state. This type of procedure is disclosed in commonly assigned U.S. Patent No. 5,342,307.

In one embodiment described therein, the wings are formed by initially pressurizing the balloon to a low pressure and centering it within tubular fixture. Movable blades which are initially in a retracted position can be simultaneously moved radially inward toward the longitudinal axis of the balloon. A vacuum is pulled through an inflation lumen to cause formation of wings. While the vacuum is maintained, the blades are returned to their retracted position and the balloon is removed from fixture. Once the winged configuration has been formed, the balloon may be heat set. Other embodiments of wing formation are also disclosed in U.S. Patent No. 5,342,307.

U.S. Patent No. 5,318,587 describes another procedure of pleating and folding expandable medical balloons. The wings may also be folded such as to form an accordion like structure.

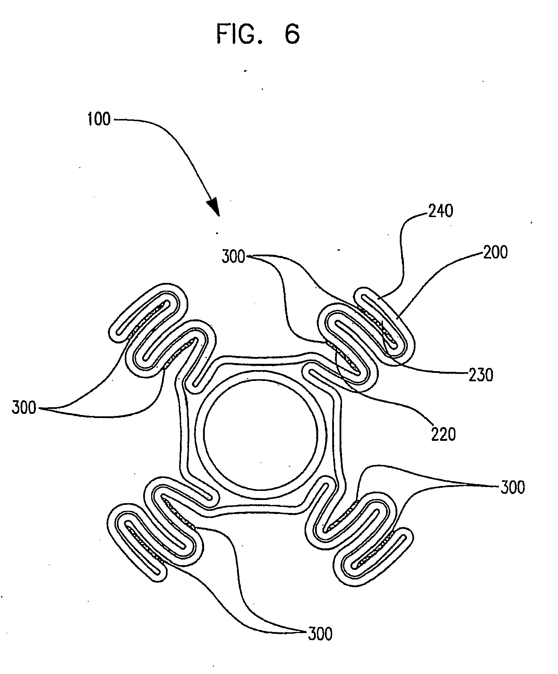

FIG. 6 is a radial cross-sectional depiction of a balloon having accordion- like folds. In this embodiment, balloon 100 is shown having four wings 200. Each wing 200 has inner surfaces 220, 230, and an outer surface 240. Inner surfaces 220, 230, may each have a lubricious surface treatment 300. Alternatively, either inner surface 220 or outer surface 230 may have a lubricious surface treatment, but not both.

In the expanded state, balloon 10 takes on a cylindrical configuration, which may be similar to that shown in FIG. 5. Balloon 100 in FIG. 6 is shown without a stent, however.

Lubricity may be imparted to the surface of the medical device using any suitable method known in the art. This can be accomplished by coating the surface with a lubricious coating. Methods of coating include, but are not limited to, pad printing, sponge coating, brushing, painting, sputtering, spraying, etc.

Examples of suitable lubricious hydrophobic materials include, but are not limited to, silicone and fluoropolymers such as polytetrafluoroethylene. These materials may be employed alone, or in combination with other materials.

Suitable hydrophilic materials include, but are not limited to, polyalkylene oxides such as polyethylene oxide, carboxylic acids such as homopolymers and copolymers of (meth) acrylic acid including polyacrylic acid homopolymer, copolymers of methylvinyl ether and maleic acid, poly(vinylpyrrolidone), poly (sodium- 4-styrenesulfonate), poly(3-hydroxybutyric acid), poly(N-alkylacrylamide), poly(vinyl alcohol), poly(ethyleneimine), polyamides, methyl cellulose, carboxymethylcellulose, polyvinylsulfonic acid, heparin, dextran, modified dextran, chondroitin sulphate, lecithin, etc., and mixtures thereof. Many of these materials are referred to in the art as hydrogel polymers.

Hydrophilic polyurethanes may be employed herein. Hydrophilic polyurethanes such as polyether polyurethanes and particularly aliphatic polyether polyurethanes may be employed herein. Hydrophilic polyurethanes are typically formed with relatively higher amounts of polyethylene oxide or polyethylene glycol. Examples of suitable hydrophilic polyurethanes are those available from Thermedics, Inc. under the tradename of TECOPHILIC®.

Examples of suitable hydrophilic lubricious polymer materials which may be employed herein are disclosed in commonly assigned U.S. Patent Nos. 5,509,899.

Interpenetrating networks (IPNs) which include hydrogel polymers may be employed herein. Such IPNs may be formed using at least one crosslinkable material and a hydrogel polymer. Suitable IPNs are disclosed in commonly assigned U.S. Patent No. 5,693,034.

Suitable crosslinkable materials include ethylenically unsaturated resins such as mono-, di- or tri- (meth)acrylates and mixtures thereof. Suitable crosslinkers of this type are found in commonly assigned copending U.S. Patent Publication US20050054744.

One suitable example of such an IPN is one including a vinyl polymer and an uncrosslinked hydrogel, in particular where polyethylene oxide (PEO) is employed as the hydrogel and the vinyl polymer is neopentyl glycol diacrylate (NPG).

A specific example is PEO hydrogel and neopentyl glycol diacrylate at a ratio of about 10:1 dissolved in a cosolvent blend of alcohol and water at a ration of 4: 1 alcohol to water. A photoinitiator such as azobis-isibutironitrilc photoinitiato can be added in minimal amounts effective to initiate NPG polymerization. Photoinitiators are known to those of skill in the art. Examples of lubricious hydro gels of this type are disclosed in commonly assigned U.S. Patent No. 5,693,034.

Another examples is PEO and ethoxylated trimethylol propane triacrylate at a ration of about 10:1, with a silane coupling agent, an acrylic flow modifier, and a small amount of a photoinitiator in a cosolvent blend of isopropyl alcohol and water at a ratio of about 4:1. This type of lubricant is disclosed in commonly assigned copending U.S. Patent Application No. 20050170071.

Another example of a suitable IPN is one using a blend of neopentyl glycol diacrylate or polyethylene glycol diacrylate and at least one hydrophilic aliphatic polyether polyurethane such as polyether polyurethanes available from Thermedics, Inc. under the tradename TECOGEL® including TECOOFL® 500 and TECOGEL® 2000.

Such IPNs are disclosed in commonly assigned copending U.S. Patent Publication Nos. US20050054774,and US20050055044, filed September 9, 2003.

Other IPNs including hydrogels are disclosed in commonly assigned U.S. Patent Nos. 6,265,016; 6,120,904; 6,080,488; 6,040,058; 6,030,656; 6,017,577; 5,919,570; 5,849,368; 5,662,960 and 5,576,072.

Another example of compositions which form hydrogels in aqueous environments when crosslinked are those disclosed in commonly assigned U.S. Patent Nos. 6,660,827; 6,534,560; 6,403,758 and 6,316,522. These are covalently crosslinkable copolymers having a bioresorbable region, a hydrophilic region and at least two crosslinkable functional groups per polymer chain.

Plasma treatment can also be employed to modify polymer coatings to impart lubriciousness, or by plasma deposition of lubricious coatings on the device surface. For example, plasma treatment can be used to strategically fluorinate the surface of the device thereby imparting lubriciousness only in predetermined specific locations on the balloon surface.

Another method of imparting lubriciousness to the surface of the medical device is to use a fluorinated sol-gel derived ceramic.

The lubricious surface treatment may be applied to the balloon at any stage during the balloon forming process, including during extrusion, by selectively coextruding a lubricious coating onto the tubular ea-trudate, for example.

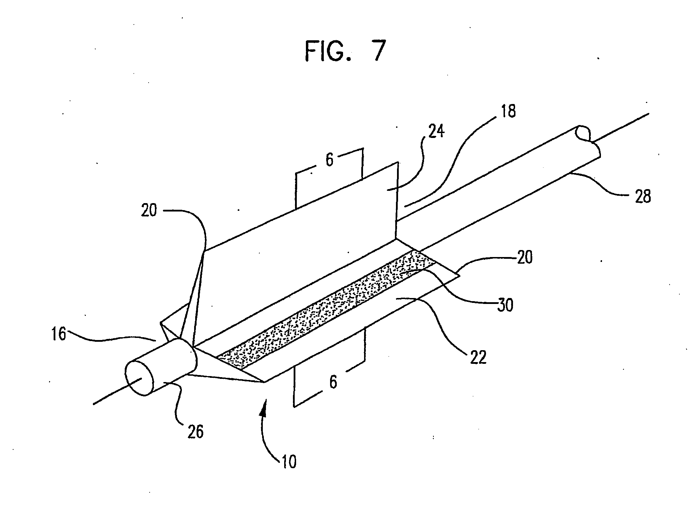

The lubricious surface treatment may also be applied to the balloon after wing formation. FIG. 7 is a perspective view of a balloon 10 is secured at its distal end 16 to an inner catheter shaft 24 and at its proximal end 18 to an outer catheter shaft 26. Balloon 10 is shown having three wings 20. Each wing 20 has a first surface 22 and a second surface 24. First surface 22 is shown having a lubricious surface treatment 30. The second surface 24 has no lubricious surface treatment.

The surface of wing 20 of balloon 10 has been treated to impart lubriciousness such that when the balloon is folded as illustrated in FIGS. 1-3, the lubricious surface does not come into contact with any implantable medical device which may be mounted on the expandable balloon.

The present invention provides an expandable medical balloon which when deflated and a stent mounted thereon, has selectively disposed lubricious surface treatment on the surface of the expandable medical balloon such that the stent has a reduced tendency to slip during delivery through a patient's body vessel to the site of deployment the stent. The balloon may then be expanded causing the stent to expand. Negative pressure may then be applied to the balloon causing it again to deflate into a second deflated configuration, different than the first. In the second deflated configuration, the lubricious surface treatment is exposed on the outer surface such that withdrawal of the deflated balloon from the stent is readily conducted, with little resistance.

The above disclosure is intended to be illustrative and not exhaustive. This description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. An expandable medical balloon (10) having a first deflated configuration comprising at least two folded wings (20), the balloon (10) having a longitudinal axis, each of said at least two folded wings (20) of said balloon (10) in said deflated configuration having a first surface (22) and a second surface (24), the first surface (22) of each of said at least two folded wings (20) being inner relative to the longitudinal axis and the second surface (24) being located outer to the longitudinal axis, at least a portion of the first surface (22) having a lubricious surface treatment and the second surface (24) having no lubricious surface treatment, **characterized in that** said lubricious surface treatment comprises fluorine atoms or fluorine comprising chemical groups covalently bonded to said surface

2. The expandable medical balloon of claim 1 wherein said expandable medical balloon (10) has at least three folded wings (20) in said deflated delivery configuration.

3. The expandable medical balloon of claim 1 wherein said expandable medical balloon (10) has at least four folded wings (20) in said deflated delivery configuration.

4. The expandable medical balloon of claim 1 wherein said expandable medical balloon (10) has at least six folded wings (20) in said deflated delivery configuration.

5. The expandable medical balloon of claim 1 further in combination with a stent, the stent mounted on said medical balloon (10), said stent in contact with said second surface (24) when said balloon is in said deflated configuration.

6. The expandable medical balloon of claim 1 wherein said lubricious surface treatment comprises a lubricious coating (30).

7. The expandable medical balloon of claim 6 wherein said lubricious coating (30) comprises a lubricious hydrophilic polymer material.

8. The expandable medical balloon of claim 7 wherein said lubricious coating (30) comprises a hydrogel.

9. The expandable medical balloon of claim 7 wherein said coating (30) comprises an interpenetrating network, said interpenetrating network comprising at least one hydrogel.

10. The expandable medical balloon of claim 1 wherein said lubricious surface treatment comprises -CF, -CF₂, -CF₃ groups or mixtures thereof covalently bonded to the surface, or branched and/or crosslinked macromolecular networks based on CF, CF₂ or CF₃ units are formed on the surface of the device.

11. The expandable medical balloon of claim 1 wherein said expendable medical balloon (10) has an expanded configuration, and a second deflated configuration after said balloon has been expanded, said balloon (10) in said second deflated configuration comprising at least two folded wings (20), each of said at least two folded wings having a first surface (22) and a second surface (24), the first surface (22) of each of said at least two folded wings (20) located inner relative to the longitudinal axis of the expandable medical balloon (10) and the second surface located outer relative to the longitudinal axis of the expandable, at least a portion of said second surface comprising said lubricious surface treatment.

12. A method of selectively treating an expandable medical balloon (10) with a lubricious surface treatment, the expandable medical balloon (10) disposed about a shaft of a catheter assembly, the method comprising the steps of:
a) applying a lubricious surface treatment to said medical balloon in predetermined locations; wherein the lubricious surface treatment comprises fluorine atoms or fluorine comprising chemical groups covalently bonded to said surface; and
b) forming at least two wings (20) in said balloon; and
c) folding said wings about said shaft;
wherein after folding said wings about said shaft, the wings have an exposed surface and a covered surface, the covered surface comprising the lubricious surface treatment.

## Patentansprüche

1. Expandierbarer medizinischer Ballon (10) mit einer ersten entleerten Konfiguration, die mindestens zwei zusammengefaltete Flügel (20) umfasst, wobei der Ballon (10) eine Längsachse aufweist, jeder der mindestens zwei zusammengefalteten Flügel (20) des Ballons (10) in der entleerten Konfiguration eine erste Oberfläche (22) und eine zweite Oberfläche (24) aufweist, die erste Oberfläche (22) von jedem der mindestens zwei zusammengefalteten Flügel (20) in Bezug auf die Längsachse weiter innen ist und die zweite Oberfläche (24) sich in Bezug auf die Längsachse weiter außen befindet, mindestens ein Teil der ersten Oberfläche (22) eine gleitfähig machende Oberflächenbehandlung aufweist und die zweite Oberfläche (24) keine gleitfähig machende Oberflächenbehandlung aufweist, **dadurch gekennzeichnet, dass** die gleitfähig machende Oberflächenbehandlung kovalent an die Oberfläche gebundene Fluoratome oder Fluor umfassende chemische Gruppen umfasst.

2. Expandierbarer medizinischer Ballon gemäß Anspruch 1, wobei der expandierbare medizinische Ballon (10) in der entleerten Freisetzungskonfiguration mindestens drei zusammengefaltete Flügel (20) hat.

3. Expandierbarer medizinischer Ballon gemäß Anspruch 1, wobei der expandierbare medizinische Ballon (10) in der entleerten Freisetzungskonfiguration mindestens vier zusammengefaltete Flügel (20) hat.

4. Expandierbarer medizinischer Ballon gemäß Anspruch 1, wobei der expandierbare medizinische Ballon (10) in der entleerten Freisetzungskonfiguration mindestens sechs zusammengefaltete Flügel (20) hat.

5. Expandierbarer medizinischer Ballon gemäß Anspruch 1, ferner in Kombination mit einem Stent, wobei der Stent auf den medizinischen Ballon (10) montiert ist und der Stent in Kontakt mit der zweiten Oberfläche (24) ist, wenn der Ballon in der entleerten Konfiguration ist.

6. Expandierbarer medizinischer Ballon gemäß Anspruch 1, wobei die gleitfähig machende Oberflächenbehandlung eine gleitfähig machende Beschichtung (30) umfasst.

7. Expandierbarer medizinischer Ballon gemäß Anspruch 6, wobei die gleitfähig machende Beschichtung (30) ein gleitfähig machendes hydrophiles Polymermaterial umfasst.

8. Expandierbarer medizinischer Ballon gemäß Anspruch 7, wobei die gleitfähig machende Beschichtung (30) ein Hydrogel umfasst.

9. Expandierbarer medizinischer Ballon gemäß Anspruch 7, wobei die Beschichtung (30) ein interpenetrierendes Netzwerk umfasst und das interpenetrierende Netzwerk mindestens ein Hydrogel umfasst.

10. Expandierbarer medizinischer Ballon gemäß Anspruch 1, wobei die gleitfähig machende Oberflächenbehandlung -CF, -CF₂, -CF₃ Gruppen oder Mischungen davon umfasst, die kovalent an die Oberfläche gebunden sind, oder verzweigte und/oder vernetzte, auf -CF, -CF₂, -CF₃- Einheiten beruhende makromolekulare Netzwerke auf der Oberfläche des Gerätes ausgebildet sind.

11. Expandierbarer medizinischer Ballon gemäß Anspruch 1, wobei der expandierbare medizinische Ballon (10) eine expandierte Konfiguration und eine zweite entleerte Konfiguration nachdem der Ballon expandiert wurde aufweist, wobei der Ballon (10) in der zweiten entleerten Konfiguration mindestens zwei zusammengefaltete Flügel (20) umfasst, jeder der mindestens zwei zusammengefalteten Flügel eine erste Oberfläche (22) und eine zweite Oberfläche (24) aufweist, die erste Oberfläche (22) von jedem der mindestens zwei zusammengefalteten Flügel (20) sich in Bezug auf die Längsachse des expandierbaren medizinischen Ballons (10) weiter innen befindet und die zweite Oberfläche sich weiter außen in Bezug auf die Längsachse des expandierbaren Ballons befindet und mindestens ein Teil der zweiten Oberfläche die gleitfähig machende Oberflächenbehandlung umfasst.

12. Verfahren zur selektiven Behandlung eines expandierbaren medizinischen Ballons (10) mit einer gleitfähig machenden Oberflächenbehandlung, wobei der expandierbare medizinische Ballon (10) über einem Schaft einer Katheterbaugruppe angeordnet ist und das Verfahren umfasst die Schritte von:
a) Aufbringen einer gleitfähig machenden Oberflächenbehandlung auf den medizinischen Ballon an vorbestimmten Stellen; wobei die gleitfähig machende Oberflächenbehandlung kovalent an die Oberfläche gebundene Fluoratome oder Fluor umfassende chemische Gruppen umfasst; und
b) Erzeugen von mindestens zwei Flügeln (20) in dem Ballon; und
c) Falten der Flügel über den Schaft;
wobei nach dem Falten der Flügel über den Schaft die Flügel eine freiliegende Oberfläche und eine bedeckte Oberfläche haben und die bedeckte Oberfläche die gleitfähig machende Oberflächenbehandlung umfasst.

## Revendications

1. Ballonnet médical expansible (10) avec une première configuration dégonflée, qui comporte au moins deux ailes repliées (20), le ballonnet (10) ayant un axe longitudinal, chacune des au moins deux ailes repliées (20) du ballonnet (10) dans la configuration dégonflée ayant une première surface (22) et une deuxième surface (24), la première surface (22) de chacune des au moins deux ailes repliées (20) étant plus à l'intérieur par rapport à l'axe longitudinal et la deuxième surface (24) se trouvant plus à l'extérieur par rapport à l'axe longitudinal, au moins une part de la première surface (22) ayant un traitement de surface lubrifiant et la deuxième surface (24) ne pas ayant un traitement de surface lubrifiant, **caractérisé en ce que** le traitement de surface lubrifiant comporte des atomes de fluorure ou des groupes chimiques comportant de la fluorure qui sont liés de façon covalente à ladite surface.

2. Ballonnet médical expansible selon la revendication 1, le ballonnet médical expansible (10) ayant au moins trois ailes repliées (20) dans la configuration de décharge dégonflée.

3. Ballonnet médical expansible selon la revendication 1, le ballonnet médical expansible (10) ayant au moins quatre ailes repliées (20) dans la configuration de décharge dégonflée

4. Ballonnet médical expansible selon la revendication 1, le ballonnet médical expansible (10) ayant au moins six ailes repliées (20) dans la configuration de décharge dégonflée.

5. Ballonnet médical expansible selon la revendication 1, en outre en combinaison avec une endoprothèse, l'endoprothèse étant montée sur le ballonnet médical (10) et l'endoprothèse étant en contact avec la deuxième surface (24) quand le ballonnet est dans la configuration dégonflée.

6. Ballonnet médical expansible selon la revendication 1, dans lequel le traitement de surface lubrifiant comporte un revêtement lubrifiant (30).

7. Ballonnet médical expansible selon la revendication 6, dans lequel le revêtement lubrifiant (30) comporte un matériau polymère hydrophile lubrifiant.

8. Ballonnet médical expansible selon la revendication 7, dans lequel le revêtement lubrifiant (30) comporte un hydrogel.

9. Ballonnet médical expansible selon la revendication 7, dans lequel le revêtement (30) comporte un réseau inter-pénétrant et le réseau inter-pénétrant comporte au moins un hydrogel.

10. Ballonnet médical expansible selon la revendication 1, le traitement de surface lubrifiant comportant des groupes -CF, -CF₂, -CF₃ ou mélanges de cela qui sont liés à la surface de façon covalente, ou des réseaux macromoléculaires branchés et/ou réticulés basés sur des unités de -CF, -CF₂, -CF₃ qui sont formés sur la surface du dispositif.

11. Ballonnet médical expansible selon la revendication 1, le ballonnet médical expansible (10) ayant une configuration épanouie et une deuxième configuration dégonflée après que le ballonnet était épanoui, le ballonnet (10) dans la deuxième configuration dégonflée comportant au moins deux ailes repliées (20), chacune des au moins deux ailes repliées ayant une première surface (22) et une deuxième surface (24), la première surface (22) de chacune des au moins deux ailes repliées (20) se trouvant plus à l'intérieur par rapport à l'axe longitudinal du ballonnet médical expansible (10) et la deuxième surface se trouvant plus à l'extérieur par rapport à l'axe longitudinal du ballonnet expansible, et au moins une part de la deuxième surface comporte le traitement de surface lubrifiant.

12. Méthode pour le traitement sélectif d'un ballonnet médical expansible (10) avec un traitement de surface lubrifiant, le ballonnet médical expansible (10) étant disposé au-dessus d'une tige d'un assemblage de cathéter, la méthode comportant les étapes de :
a) appliquer un traitement de surface lubrifiant sur le ballonnet médical dans des parties prédéterminées; le traitement de surface lubrifiant comportant des atomes de fluorure ou des groupes chimiques comportant de la fluorure qui sont liés de façon covalente à ladite surface; et
b) former au moins deux ailes (20) dans le ballonnet; et
c) plier les ailes au-dessus de la tige;
après le pliage des ailes au-dessus de la tige les ailes ayant une surface exposée et une surface recouverte, la surface recouvert comportant le traitement de surface lubrifiant.
